(19) Europäisches Patentamt
European Patent Office
Office européen des brevets

(11) Veröffentlichungsnummer: **0 018 568**
**B1**

# (12) EUROPÄISCHE PATENTSCHRIFT

(45) Veröffentlichungstag der Patentschrift: **19.01.83**

(51) Int. Cl.³: **C 07 D 233/58**

(21) Anmeldenummer: **80102130.4**

(22) Anmeldetag: **21.04.80**

## (54) Verfahren zur Herstellung von Imidazolen.

(30) Priorität: **28.04.79 DE 2917381**

(43) Veröffentlichungstag der Anmeldung: **12.11.80 Patentblatt 80/23**

(45) Bekanntmachung des Hinweises auf die Patenterteilung: **19.01.83 Patentblatt 83/3**

(84) Benannte Vertragsstaaten: **BE CH DE FR GB IT LI**

(56) Entgegenhaltungen:
**CA-A-1 075 689**
**DE-A-2 360 175**
**DE-A-2 614 412**
**US-A-3 715 365**
**Chemical Abstracts Band 88, Nr. 5, 30. Januar 1978 Columbus, Ohio, USA J. J. BALDWIN »Substituted imidazoles« Seite 505, Spalte 1, Abstract Nr. 37802 f**
**K. HOFMANN »The Chemistry of Heterocyclic Compounds, Imidazole« Teil I, 1953, INTERSCIENCE PUBLISHERS, New York Seiten 33 bis 45**

(73) Patentinhaber: **BASF Aktiengesellschaft, Carl-Bosch-Strasse 38, D-6700 Ludwigshafen (DE)**

(72) Erfinder: **Eberhard, Peter, Dr., Ungsteiner Strasse 2, D-6700 Ludwigshafen (DE)**
Erfinder: **Hupfer, Leopold, Dr., Waltershoehe 3, D-6701 Friedelsheim (DE)**
Erfinder: **Graf, Fritz, Dr., Im Rothschild 33, D-6720 Speyer (DE)**
Erfinder: **Schulte, Wolfgang, Dr., Barbarossastrasse 9, D-6733 Hassloch (DE)**

**EP 0 018 568 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Imidazolen

Die Erfindung betrifft ein Verfahren zur Herstellung von Imidazolen durch Umsetzung von acetalisierten $\alpha$-Dicarbonylverbindungen mit Ammoniak und/oder Ammoniumsalzen und Aldehyden in Gegenwart starker Säuren und nachfolgende Extraktion der gebildeten Imidazole durch organische Lösungsmittel.

Imidazole lassen sich beispielsweise durch Kondensation von Glyoxal mit Ammoniak und Aldehyden herstellen (Klaus Hoffmann, The Chemistry of Heterocyclic Compounds, Imidazole, Part I (1953), Seiten 33 bis 45). Nach diesem Verfahren werden nur mäßige Ausbeuten erhalten. Durch Zusatz von organischen Lösungsmitteln und starke Verkürzung der Verweilzeiten läßt sich die Ausbeute verbessern (DE-OS 2 360 175). Die Synthese versagt jedoch, wenn man anstelle von Glyoxal Methylglyoxal verwendet (Tadashi Sasaki, Chemical Abstracts, Band 53 (1959), 113 92). Nach den Angaben der US-PS 3 715 365 wird 4-Methylimidazol aus Brenztraubenaldehyd, Formaldehyd, Ammoniak bzw. Ammoniumsulfat in einer Ausbeute von 59% und einer Reinheit von ca. 77% gewonnen, wenn das Reaktionsgemisch entsprechend einer umständlichen Aufarbeitungsmethode mit Calciumhydroxid versetzt und der Überschuß Calciumhydroxid mit Kohlendioxid beseitigt wird.

In der CA-A-1 075 689 wird ein Verfahren zur Herstellung von Imidazolen beschrieben, bei dem Aldehyde mit einer Glyoxalverbindung und Ammoniak bei Temperaturen bis 100°C kondensiert werden. Man kann dabei auch von einem Acetal der Glyoxalverbindung ausgehen, das zunächst durch Behandlung mit einer starken Säure wie Schwefelsäure hydrolysiert wird.

Aus der DE-A-2 616 412 ist ein Verfahren zur Herstellung von Imidazolen durch Kondensation von Glyoxal und einem Aldehyd und Ammoniak bekannt, bei dem man das Glyoxal in Form seiner Bisulfitverbindung anwendet und die Kondensation bei einem pH-Wert zwischen 6 und 8 durchführt.

Auch bei diesen bekannten Verfahren läßt die Reinheit der Imidazole zu wünschen übrig.

Da Imidazole als Zwischenprodukte z. B. für die Herstellung von Heilmitteln zunehmendes technisches Interesse gefunden haben, war nach einem Verfahren zu suchen, nach dem Imidazole technisch vorteilhaft und mit verbesserten Ausbeuten und Reinheiten hergestellt werden können.

Es wurde nun gefunden, daß man Imidazole der Formel

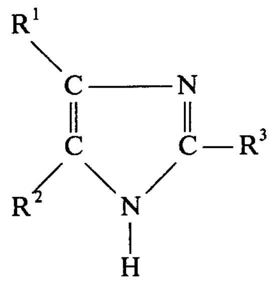

(I)

in der $R^1$ und $R^2$ Wasserstoffatome oder aliphatische, aromatische, araliphatische oder cycloaliphatische Reste und $R^3$ ein Wasserstoffatom oder einen aliphatischen, aromatischen oder heterocyclischen Rest bedeuten, besonders vorteilhaft herstellen kann, wenn man acetalisierte $\alpha$-Dicarbonylverbindungen der Formeln

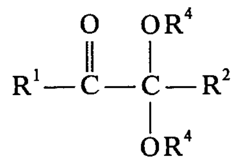

oder

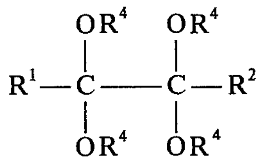

(II) (III)

in denen $R^4$ für einen Alkylrest steht, in Gegenwart von Wasser bei Temperaturen bis 100°C mit einer starken Säure und einem Aldehyd der Formel $R^3-CHO$ (IV) behandelt, zu dem sauren Gemisch Ammoniak und/oder Ammoniumsalze so gibt, daß der pH-Wert des Gemisches zwischen 1 und 4 liegt, das Reaktionsgemisch auf 20 bis 140°C erhitzt, durch Zugabe von Basen einen pH-Wert von über 8,5 einstellt und das gebildete Imidazol aus der wäßrigen Reaktionslösung mit einem organischen Lösungsmittel extrahiert.

Die als Ausgangsverbindungen verwendeten Acetale der Formeln II und III enthalten als Reste $R^1$ und $R^2$ Wasserstoffatome oder aliphatische Reste, wie Alkylreste mit 1 bis 18 C-Atomen, die noch Hydroxylgruppen enthalten können, wie Methyl-, Äthyl-, Propyl-, oder Butylgruppen oder Hydroxymethylgruppen, Carboxylgruppen, die mit Alkoholen oder Phenolen verestert sein können,

wie Methyl-, Äthyl- oder Phenylestergruppen, aromatische Reste, wie Phenylreste, araliphatische Reste, wie Benzylreste oder cycloaliphatische Reste, wie Cyclohexylgruppen.

$R^4$ steht für einen Alkylrest, der z. B. 1 bis 18, vorzugsweise 1 bis 4 C-Atome enthalten kann, insbesondere jedoch für den Methylrest. Beispielsweise seien folgende Acetale genannt: Glyoxaltetramethylacetal, Methylglyoxaldimethylacetal, Phenylglyoxaldimethylacetal und Redukton.

Als $R^3$-Reste kommen z. B. Wasserstoffatome, Alkylgruppen mit 1 bis 4 C-Atomen, die noch Hydroxylgruppen, Halogenatome oder Alkoxygruppen enthalten können, wie Methyl-, Äthyl-, Propyl- oder Butylgruppen, Chlormethyl- oder Methoxymethylgruppen, Alkylengruppen, wie Äthylengruppen, Phenylreste, die Alkyl-, Alkoxy-, Alkylgruppen oder Halogenatome tragen können oder heterocyclische Reste, wie gegebenenfalls substituierte Furylreste, in Betracht.

Aldehyde der Formel IV sind z. B. Formaldehyd, Acetaldehyd, Propionaldehyd, Chloracetaldehyd, Methoxyacetaldehyd, n-Butyraldehyd, Isobutyraldehyd, Benzaldehyde, wie Hydroxy-, Methoxy-, Chlorbenzaldehyd oder Tolylaldehyd. Es kommen auch ungesättigte Aldehyde, wie 3,3-Dimethylacrolein, Furfurole, wie Furfurol, 5-Hydroxymethylfurfurol, 5-Methylfurfurol und Glyoxylsäure in Betracht.

Als starke Säuren verwendet man Mineralsäuren, z. B. Schwefelsäure oder Halogenwasserstoffsäuren, wie Salzsäure, Bromwasserstoffsäure und Perchlorsäure. Es kommen aber auch andere starke Säuren, wie Ameisensäure oder Sulfonsäuren, wie Benzolsulfonsäure, in Betracht. Ferner sind auch saure Ionenaustauscher geeignet. Schwefelsäure ist bevorzugt.

Nach dem neuen Verfahren werden die Acetale der Formeln II oder III in Gegenwart von Wasser bei Temperaturen bis 100°C mit einer starken Säure und einem Aldehyd der Formel $R^3-CHO$ (IV) behandelt. Werden schwer wasserlösliche Acetale verwendet, so kann man mit Wasser mischbare Lösungsmittel, wie Essigsäure, Ameisensäure oder Alkohole, wie Methanol oder Äthanol, Dioxan, Sulfolan oder Tetrahydrofuran zusetzen.

Die Behandlung mit Säure und Aldehyd, bei der im Reaktionsgemisch der Alkohol der Formel $R^4OH$ frei wird, ist nach etwa 0,5 bis 5 Stunden beendet. Der Aldehyd muß nicht zu Beginn der Säurebehandlung zugegeben werden. Man kann ihn auch während oder auch am Schluß der Säurebehandlung zugeben.

Vorteilhaft ist es, wenn man während der Säurebehandlung und vor der Zugabe von Ammoniak und/oder Ammoniumsalzen aus dem stark sauren Reaktionsgemisch ein Gemisch aus Wasser und Alkohol ($R^4OH$) abdestilliert. Bei dieser Ausführungsform des Verfahrens ist es günstig, wenn man den Aldehyd erst nach dem Abdestillieren des Gemisches aus Wasser und Alkohol zugibt.

Die bei dieser bevorzugten Arbeitsweise eingesetzten Ausgangsgemische enthalten z. B. 5 bis 60 Gewichtsprozent Acetal, 30 bis 90 Gewichtsprozent Wasser, 0 bis 50 Gewichtsprozent Lösungsmittel und 0,5 bis 20 Gewichtsprozent Mineralsäure.

Man destilliert aus diesen stark sauren Reaktionsgemischen ein Gemisch aus Wasser und dem Alkohol ab, indem man das Reaktionsgemisch unter Normaldruck oder vermindertem Druck auf 50 bis 100°C erhitzt. Bei Verwendung von Methylacetalen destilliert das Methanol/Wasser-Gemisch bei einer Sumpftemperatur von 50 bis 100°C und bei einer Übergangstemperatur von 40 bis 95°C ab. Dieser Verfahrensschritt ist beendet, wenn der Alkoholgehalt im Rückstand kleiner ist als 0,01 Mol pro Mol Dicarbonylverbindung.

Man gibt nun zu dem stark sauren Rückstand den Aldehyd sowie Ammoniak und/oder ein Ammoniumsalz, wie Ammoniumsulfat, Ammoniumchlorid, Ammoniumbromid, Ammoniumsulfonat oder ammoniumbeladene Ionenaustauscher. Dabei wird das Ammoniak gasförmig eingeleitet oder als wäßrige Lösung zugegeben. Wesentlich ist hierbei, daß der pH-Wert im Reaktionsgemisch bei Werten zwischen 1 und 4 liegt.

Die Aldehyde werden in mindestens stöchiometrischer Menge und Ammoniak in höchstens stöchiometrischer Menge eingesetzt. Die in den Reaktionsgemischen enthaltenen Mengen an Ammoniumsalzen betragen 0,1 bis 3 Mol, bezogen auf ein Mol Acetal. Diese Mengen werden entweder direkt zugegeben oder aus einem entsprechenden Überschuß Mineralsäure und Ammoniak im Reaktionsgemisch gebildet.

Das Reaktionsgemisch wird zur Bildung der Imidazole auf 20 bis 140°C erhitzt. Die Reaktionszeiten betragen für diese zweite Stufe des Verfahrens je nach der gewählten Temperatur und Ausgangsstoffe 0,1 bis 12 Stunden. In den meisten Fällen genügen 0,5 bis 4 Stunden.

Man arbeitet auf, indem man im Reaktionsgemisch durch Zugabe von Basen einen pH-Wert von über 8,5 einstellt. Als Basen sind solche Verbindungen geeignet, die stärker basisch sind als das hergestellte Imidazol, wie Ammoniak, Natronlauge oder Kalilauge. Zweckmäßigerweise stellt man den pH-Wert des Reaktionsgemisches auf Werte von 9 bis 13. Das gebildete Imidazol wird dann mit einem organischen Lösungsmittel aus der wäßrigen Lösung, die gegebenenfalls vorher eingeengt wird, extrahiert. Für diese Extraktion geeignete Lösungsmittel sind z. B. Methylenchlorid, Äther, Tetrachlorkohlenstoff, Benzonitril und Dibutylformamid.

Die durch Abdampfen des Extraktionsmittels erhältlichen Rohimidazole können auf an sich übliche Weise, z. B. durch einfache Destillation, gereinigt werden.

Nach dem neuen Verfahren werden die Imidazole in hoher Ausbeute und Reinheit erhalten.

## Beispiel 1

59 Teile Methylglyoxaldimethylacetal, 280 Teile Wasser und 10 Teile konzentrierte Schwefelsäure werden bei Raumtemperatur solange gerührt, bis eine homogene Mischung entstanden ist. Dann werden unter Erwärmen bis zu einer Sumpftemperatur von 96° C und einer Übergangstemperatur von 85 bis 95° C bei Normaldruck etwa 60 Teile eines Gemisches aus Methanol und Wasser möglichst rasch und schonend abdestilliert. Dem Rückstand (pH-Wert ca. 0,5) setzt man 38 Teile 40%igen Formaldehyd und 150 Teile Ammoniumsulfat zu. Die Mischung wird 4 Stunden auf 90° C erwärmt, wobei sich ein pH-Wert von ca. 1 ergibt. Dann wird durch Zugabe von 100 Teilen 25%igem Ammoniakwasser ein pH-Wert von ca. 10 eingestellt und das Reaktionsgemisch mit Methylenchlorid ausgeschüttet. Man destilliert das Extraktionsmittel ab und erhält 26 Teile Rohimidazol. Durch Einengen des Reaktionsgemisches, erneutes Zusetzen von 50 Teilen 25%igem wäßrigem Ammoniak und wiederholtes Ausschütteln mit Methylenchlorid erhält man nochmals 7 Teile Rohimidazol. Die Rohimidazol-Fraktionen werden vereinigt und im Vakuum bei 18 Torr/159 bis 163° C ohne Kolonne destilliert. Dabei gewinnt man 27 Teile kristallines 4-Methylimidazol (Schmelzpunkt 47 bis 48° C) mit einer Reinheit von 95% entsprechend einer Ausbeute von 63% der Theorie neben 4 Teilen Destillationsrückstand. Die wäßrige, ausgeschüttelte, ammoniumsulfathaltige Raffinatphase kann nach dem Ansäuern wieder eingesetzt werden.

## Beispiel 2

59 Teile Methylglyoxaldimethylacetal, 280 Teile Wasser und 10 Teile konzentrierte Schwefelsäure werden wie in Beispiel 1 beschrieben nach dem Abdestillieren von Wasser und Methanol mit 38 Teilen 40%igem Formaldehyd und 75 Teilen Ammoniumsulfat zunächst bei pH = 1,0 bis 1,5 und bei 90° C 2 Stunden zur Reaktion gebracht. Danach werden 20 Teile 25%ige Ammoniaklösung so zudosiert, daß der pH-Wert am Ende bei 2,0 liegt. Nach weiterem zweistündigen Erwärmen auf 90° C wird das Reaktionsgemisch wie im Beispiel 1 angegeben mit 75 Teilen Ammoniakwasser alkalisch gestellt, mit Methylenchlorid ausgeschüttelt, auf etwa die Hälfte eingeengt und mit 50 Teilen Ammoniakwasser nochmals alkalisch gestellt und erneut mit Methylenchlorid ausgeschüttelt. Man isoliert 13 Teile und 18 Teile Rohimidazol, die bei 18 Torr/155 bis 159° C in einer einfachen Destillationsapparatur 24 Teile 4-Methylimidazol mit einer Reinheit >99%, entsprechend 58% der Theorie, liefern. Die Rückstandsmenge beträgt 4 Teile, in den 1 bis 3 Teilen Vorlauf sind noch ca. 80% 4-Methylimidazol enthalten.

## Beispiel 3

118 Teile Methylglyoxaldimethylacetal, 236 Teile Wasser und 10 Teile konzentrierte Schwefelsäure werden vermischt. 88 Teile Methanol/Wasser werden bis zu Sumpftemperaturen von 95° C und Übergangstemperaturen von 92° C abdestilliert. Zu dem Rückstand (280 Teile) gibt man nach dem Abkühlen auf Raumtemperatur 76 Teile 40%igen wäßrigen Formaldehyd sowie 132 Teile Ammoniumsulfat und erhitzt die Mischung, die einen pH-Wert von ca. 1,5 aufweist, 4 Stunden auf 90° C. Nach dem Abkühlen auf 30 bis 35° C wird durch Zugabe von 310 Teilen 25%ige Natronlauge der pH-Wert von 1 auf 10 bis 11 angehoben. Durch Extraktion mit Methylenchlorid erhält man 53 Teile rohes 4-Methylimidazol und daraus durch Destillation bei 17 Torr/153 bis 155° C 48 Teile reines 4-Methylimidazol in einer Ausbeute von 59% der Theorie mit einem Schmelzpunkt von 47 bis 49° C und einer Reinheit größer als 95%. Die Rückstandsmenge beträgt 4 Teile.

## Beispiel 4

Aus dem Gemisch von 59 Teilen Methylglyoxaldimethylacetal, 250 Teilen Wasser, 10 Teilen konzentrierter Schwefelsäure wird wie in den Beispielen 1 bis 3 angegeben ein Gemisch aus Methanol und Wasser abdestilliert. Zum Destillationsrückstand werden 75 Teile Ammoniumsulfat und 38 Teile 40%ige Formaldehydlösung gegeben. Der pH-Wert des Gemisches beträgt 1. Nach 5stündigem Erwärmen auf 55 bis 60° C wird abgekühlt und mit 210 Teilen 30%iger Natronlauge der pH-Wert auf 13 angehoben. Man destilliert 100 Teile Wasser ab, schüttelt mit Methylenchlorid aus und entfernt das Extraktionsmittel. Als Rückstand verbleiben 35 Teile Rohimidazol, aus denen nach einfacher Vakuumdestillation bei 110 bis 128° C/3 Torr 30 Teile 4-Methylimidazol mit einem Gehalt von 93,4% und einem Schmelzpunkt von 48 bis 50° C, entsprechend einer Ausbeute von 68% der Theorie, erhalten werden.

Beispiel 5

Aus dem Gemisch von 118 Teilen Methylglyoxaldimethylacetal, 500 Teilen Wasser und 20 Teilen Schwefelsäure wird wie in den anderen Beispielen beschrieben Methanol/Wasser abdestilliert. Zum Rückstand werden 44 Teile Acetaldehyd und 132 Teile Ammoniumsulfat gegeben. Das Reaktionsgemisch, das einen pH-Wert von ca. 2 aufweist, wird 7 Stunden auf 85°C erhitzt. Dann stellt man mit 30%iger Natronlauge auf pH = 12,5 ein, schüttelt mit Methylenchlorid aus, engt ein und destilliert den Extraktionsrückstand im Vakuum. Bei 130 bis 135°C/2 Torr gehen 40 Teile 2,4-Dimethylimidazol über (Ausbeute 41% der Theorie, Schmelzpunkt 89 bis 90°C, Reinheit 99%).

Vergleichsbeispiel

Das Reaktionsgemisch wird gemäß der US-PS 3 725 365 aufgearbeitet, indem man Calciumhydroxid zugibt und mit Kohlendioxid begast.

59 Teile Methylglyoxaldimethylacetal, 300 Teile Wasser und 10 Teile konzentrierte Schwefelsäure werden vermischt und 0,5 Stunden bei Raumtemperatur bis zur Bildung einer homogenen Lösung gerührt. Aus dieser Lösung werden bei 100°C Sumpftemperatur und 85 bis 95°C Übergangstemperatur 65 Teile Methanol/Wasser abdestilliert. Nach Zugabe von 35 Teilen Ammoniumsulfat und 41 Teilen 40%iger Formaldehydlösung wird die Mischung (pH-Wert = 1,0) auf 90°C erwärmt. Innerhalb von 1,5 Stunden gibt man 45 Teile 25%iges Ammoniakwasser hinzu und rührt das Gemisch 4 Stunden bei 90°C (pH-Wert = 4,5).

Durch Hinzufügen von 30 Teilen Calciumhydroxid und 1stündiges Erhitzen auf 90°C wird der pH-Wert auf 10 gestellt. Dann wird mit Kohlendioxid bei 90°C 3 Stunden begast. Man filtriert vom Niederschlag ab, wäscht dreimal mit heißem Wasser und engt die wäßrige Lösung im Vakuum ein. Dabei gehen 268 Teile bei 35 bis 53°C und 28 bis 105 Torr über. Es verbleibt ein Rückstand (54 Teile), aus dem auch bei 3 Torr kein Destillat übergeht, vielmehr verkohlt der Rückstand.

**Patentansprüche**

1. Verfahren zur Herstellung von Imidazolen der Formel

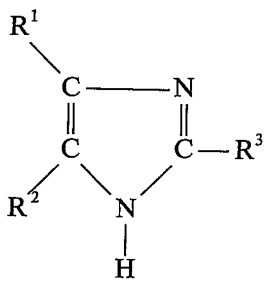

(I)

in der $R^1$ und $R^2$ Wasserstoffatome oder aliphatische, aromatische, araliphatische oder cycloaliphatische Reste und $R^3$ ein Wasserstoffatom oder einen aliphatischen, aromatischen oder heterocyclischen Rest bedeuten, dadurch gekennzeichnet, daß man acetalisierte $\alpha$-Dicarbonylverbindungen der Formeln

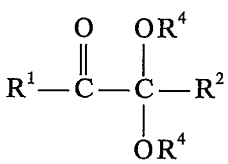

oder

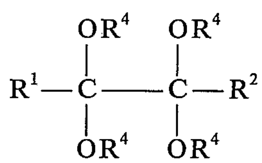

(II) (III)

in denen $R^4$ für einen Alkylrest steht, in Gegenwart von Wasser bei Temperaturen bis 100°C mit einer starken Säure und einem Aldehyd der Formel $R^3$—CHO (IV) behandelt, zu dem sauren Gemisch Ammoniak und/oder Ammoniumsalze so gibt, daß der pH-Wert des Gemisches zwischen 1 und 4 liegt, das Reaktionsgemisch auf 20 bis 140°C erhitzt, durch Zugabe von Basen einen pH-Wert von über 8,5 einstellt und das gebildete Imidazol aus der wäßrigen Reaktionslösung mit einem organischen Lösungsmittel extrahiert.

2. Verfahren nach Anspruch 1, dadurch gekennzeichnet, daß man aus dem stark sauren Gemisch vor

der Zugabe von Ammoniak und/oder Ammoniumsalzen ein Gemisch aus Wasser und Alkohol ($R^4OH$) abdestilliert.

3. Verfahren nach Anspruch 2, dadurch gekennzeichnet, daß man den Aldehyd erst nach dem Abdestillieren des Gemisches aus Wasser und Alkohol zugibt.

**Claims**

1. Process for the preparation of imidazoles of the formula

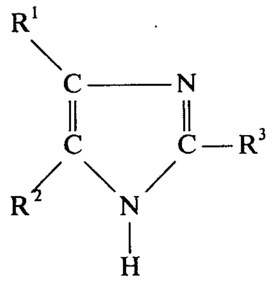

(I)

in which $R^1$ and $R^2$ denote hydrogen atoms or aliphatic, aromatic, araliphatic or cycloaliphatic radicals and $R^3$ denotes a hydrogen atom or an aliphatic, aromatic or heterocyclic radical, wherein acetalized $\alpha$-dicarbonyl compounds of the formulae

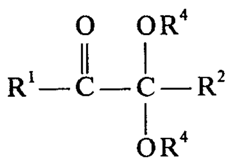 or 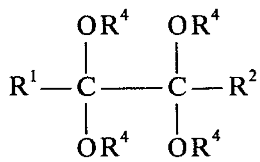

(II) (III)

in which $R^4$ represents an alkyl radical are treated with a strong acid and an aldehyde of the formula $R^3-CHO$ (IV) in the presence of water at temperatures of up to 100°C, ammonia and/or ammonium salts are added to the acid mixture such that the pH value of the mixture is between 1 and 4, the reaction mixture is heated to from 20 to 140°C, the pH value is brought to above 8.5 by addition of bases, and the imidazole formed is extracted from the aqueous reaction solution with an organic solvent.

2. Process according to claim 1, wherein a mixture of water and alcohol ($R^4OH$) is distilled off from the strongly acid mixture before the addition of ammonia and/or ammonium salts.

3. Process according to claim 2, wherein the aldehyde is added only after the mixture of water and alcohol has been distilled off.

**Revendications**

1. Procédé de préparation d'imidazoles de la formule

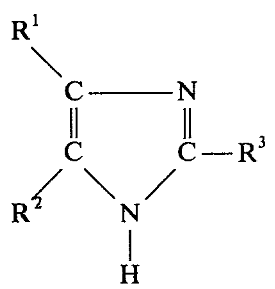

(I)

dans laquelle $R^1$ et $R^2$ désignent des atomes d'hydrogène ou des restes aliphatiques, aromatiques, araliphatiques ou cycloaliphatiques et $R^3$ représente un atome d'hydrogène ou un reste aliphatique, aromatique ou hétérocyclique, caractérisé en ce que l'on traite des composés $\alpha$-dicarbonylés acétalisés de l'une des formules

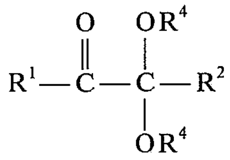 et 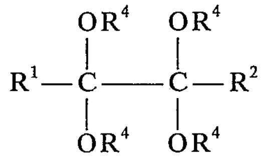

(II) (III)

dans lesquelles $R^4$ désigne un radical alcoyle, à des températures pouvant aller jusqu'à 100° C et en présence d'eau avec un acide fort et un aldéhyde de la formule $R^3-CHO$ (IV), on ajoute au mélange acide des proportions telles d'ammoniac et (ou) de sels d'ammonium que le pH du mélange se situe entre 1 et 4, on chauffe le mélange réactionnel entre 20 et 140° C, puis on ajuste le pH par addition de bases à une valeur supérieure à 8,5 et on extrait l'imidazole formé de la solution réactionnelle aqueuse à l'aide d'un solvant organique.

2. Procédé suivant la revendication 1, caractérisé en ce que, préalablement à l'addition d'ammoniac et(ou) de sels d'ammonium au mélange fortement acide, on élimine de celui-ci un mélange d'eau et d'alcool ($R^4OH$) par distillation.

3. Procédé suivant la revendication 2, caractérisé en ce que l'aldéhyde n'est ajouté qu'après l'extraction par distillation du mélange d'eau et d'alcool.